# EUROPEAN PATENT SPECIFICATION

(11) **EP 3 049 433 B1**
(45) Date of publication and mention of the grant of the patent: **07.11.2018**
(21) Application number: 14727636.4
(22) Date of filing: 15.04.2014
(51) Int. Cl.: C07K 14/505, C07K 14/54, C07K 14/535, C07K 14/475

(54) **FUSED PROTEINS OF GRANULOCYTE COLONY-STIMULATING FACTOR WITH OTHER PARTNERS OF GROWTH FACTOR, PREFERABLY WITH STEM CELL FACTOR, AND METHOD OF PREPARATION THEREOF**
FUSIONIERTE PROTEINE DES GRANULOZYTKOLONIE-STIMULIERENDEN FAKTORS MIT ANDEREN PARTNERN DES WACHSTUMSFAKTORS, VORZUGSWEISE MIT STAMMZELLENFAKTOR, UND VERFAHREN ZUR HERSTELLUNG DAVON
PROTÉINES DE FUSION CONSTITUÉES D'UN FACTEUR DE STIMULATION DES COLONIES GRANULOCYTAIRES AVEC D'AUTRES PARTENAIRES DE TYPE FACTEUR DE CROISSANCE, DE PRÉFÉRENCE AVEC UN FACTEUR DE CROISSANCE DE CELLULES SOUCHES, ET LEUR PROCÉDÉ DE PRÉPARATION

(30) Priority: 27.09.2013 LT 2013104
(43) Date of publication of application: 03.08.2016
(73) Proprietor: UAB Profarma, 02241 Vilnius (LT)
(72) Inventor: PESLIAKAS, Jonas Henrikas, LT-04001 Vilnius (LT); PLECKAITYTE, Milda, LT-10007 Vilnius (LT); MISTINIENE, Edita, LT-02011 Vilnius (LT); ZVIRBLYTE, Gitana, LT-03120 Vilnius (LT); PETREIKYTE, Indre, LT-03121 Vilnius (LT); LEIVA, Audrius, LT-11306 Vilnius (LT); STIRKE, Arunas, LT-05221 Vilnius (LT); ZVIRBLIS, Gintautas, LT-06111 Vilnius (LT)
(74) Representative: Gerasimovic, Liudmila
(86) International application number: PCT/LT2014/000006
(87) International publication number: WO 2015/047062

(56) References cited:
- WO-A1-01/03737
- WO-A1-92/04455
- WO-A1-92/06116
- WO-A1-2005/044296
- WO-A1-2008/006899
- WO-A1-2011/147319
- WO-A1-2013/022328
- CN-C- 100 336 907
- US-A1- 2007 081 979

## Description

### Field of the invention

This invention belongs to the field of protein biotechnology and is designated for production of protein of therapeutic value. Actually, the present invention provides the possibility of preparation of genetically fused heterodimers of granulocyte colony-stimulating factor (G-CSF) with other partners of hematopoietic growth factors acting synergistically with G-CSF, which dimer constructions are characterized by longer circulation half-life comparing with each monomer protein in separate state. More particularly, the present invention relates to heterodimer of granulocyte colony-stimulating factor comprising monomer unit of granulocyte colony-stimulating factor connected genetically *via* the linker of defined length to the monomer unit of stem cell factor (SCF), as well as with efficient isolation and purification of biologically active G-CSF - SCF heterodimer protein produced. The present invention also relates to a synthetic gene coding for heterodimer form of G-CSF with SCF containing linker of defined length between both monomer units for the expression in host cells.

### Background of the invention

In the present application the term "protein of therapeutic value" means pharmacologically active protein produced through genetic engineering, including mammalian antibodies, blood product substitutes, vaccines, hormones, cytokines. To be efficacious, protein drugs are used in concentrations and under conditions which differ markedly from their native partners and this may lead to undesirable effects *in vivo.* To avoid or minimize toxicity and to increase efficacy both the physicochemical and biological properties of proteins are being altered generally by some form of protein modification e.g., covalent conjugation with other macromolecules; antibody binding, mutagenesis and/or glycosylation.

The term "hematopoietic growth factor" designates one of a group of proteins, such as erythropoietin, interleukins, and colony-stimulating factors, which promote the proliferation of blood cells.

The term "biologically active protein" means the protein molecule which exhibits the same detectable biological activity as the respective naturally occurring protein.

The term "monomer unit" (in, e.g. dimer proteins) means a polypeptide chain of naturally occurring biologically active protein.

The term "heterodimer" stands for a linear polypeptide chain comprising two different biologically active polypeptide units which are connected to each other *via* peptide linker sequence in such a manner that possibility of intermolecular disulfide bonds between polypeptide chains is minimal.

The term "genetically fused" means that heterodimer protein is obtained using recombinant DNA methodology: DNA fragment, constructed of two genes, encoding monomer proteins via linker DNA sequence, are inserted into the vector for expression of the protein in the selected cell line. The resulting recombinant protein, consisting from monomer proteins connected with linker peptide, (which protein could be isolated by applying special purification procedures) clearly differs from both "chemically conjugated" protein that is obtained by chemical joining of two or more monomer proteins using specific chemical methods and from "chemically modified" protein that is obtained by chemical modification of monomer protein using chemical agents or polymer residues.

The term "synergistic effect" or "synergy" means such joint action of two drugs, where one thereof supplements or enhances the action of the other to produce an efficiency greater than that obtained with equivalent quantity of either one of the drugs or to produce effects that could not be obtained with any safe quantity of either or both preparations. Acting in synergy two or more pharmaceuticals are able to produce greater effect, than the sum of individual effects thereof.

Over the last decades the use of therapeutic proteins produced by recombinant DNA technique have expanded remarkably, as well as new therapeutic use by using combination therapy of several proteins.
It has been known for a more than three decades that hematopoiesis is controlled by very specific factors, acting the early cells in the hematopoietic system to convert them into functional cells. The isolation from natural sources, purification and cloning of these factors have discovered a new class of therapeutic agents, including the colony-stimulating factors. One of them, the granulocyte colony-stimulating factor (G-CSF), specifically acts on neutrophils, the body's major defense system against infections. The bacterially produced form thereof under the generic name "Filgrastim"(r-metHuG-CSF) has opened wide range of therapeutical uses for ameliorating neutropenia, one of the dominant side effects of cancer chemotherapy. Its use has allowed to reduce the risk of infections for cancer patients. Besides chemotherapy-induced neutropenia, Filgrastim has been approved for the treatment of myelosuppression after bone marrow transplantation, severe chronic neutropenia, acute leukemia, aplastic anemia, myelodysplastic syndromes. Filgrastim is also known for use in transplantation of peripheral blood progenitor cells. The range of G-CSF indications is expanding and combination therapeutic effect thereof with others cytokines are extensively investigated discovering new areas of potential clinical application of G-CSF.
Currently various G-CSF application areas are known:
- besides its major indication in treating of chemotherapy-induced neutropenia, it has been suggested as a novel, noninvasive therapeutic agent for the treatment of Alzheimer's disease [Kuen-Jer Tsai et al, G-CSF rescues the memory impairment of animal models of Alzheimer's disease. JEM. The Rockefeller University Press ,Vol. 204, No. 6, June 11, 1273-1280, 2007];
- the use of G-CSF polypeptide as well together with additional growth factors, including SCF, is described for prevention of myocardial infarction or for treating of the latter and other ischemic disorders, especially in treatment of acute myocardial infarction [WO2005044296 (prior. 2003-10-27)];
- It has been published that G-CSF mobilized progenitor cells are not only capable of tissue differentiation but are also likely to regenerate the myocardium, resulting in improved cardiac function [Tsung-Ming Lee et al. Granulocyte colony-stimulating factor increases sympathetic reinnervation and the arrhythmogenic response to programmed electrical stimulation after myocardial infarction in rats. Am J Physiol Heart Circ Physiol 297, H512-H522, 2009];
- G-CSF is a potential clinical treatment option for both neurodegenerative diseases and glaucoma [T. Frank et al. Both systemic and local application of Granulocyte-colony stimulating factor (G-CSF) is neuroprotective after retinal ganglion cell axotomy. BMC Neuroscience 2009];
- the use of G-CSF or fragments thereof in pharmaceutical composition for treating of organs dysfunction caused by ischemia is disclosed in patients subjected to a surgical or other interventional procedure in order to improve blood flow and/or to induce revascularization [WO2005049062 / EP1689420 (prior. 2003-10-27)];
- G-CSF was found to promote brain neurogenesis in mature animals after stroke [Aurel Popa-Wagner et al, Effects of granulocyte-colony stimulating factor after stroke in aged rats Stroke 2010, 41:1027-1031];
- G-CSF can increase the therapeutic efficacy of BMMCs transplantation in an experimental mouse model of cerebral ischemia [Zhang et al. Granulocyte colony-stimulating factor increases the therapeutic efficacy of bone marrow mononuclear cell transplantation in cerebral ischemia in mice. BMC Neuroscience 2011, 12:61];
- disclosed is the use of combined pharmaceutical preparation of G-CSF and placental growth factor in mobilization of blood stem cells [WO2002014023/EP1660115 (prior. 2003-07-29)].

### Combination therapy

Hematopoietic growth factors have redundant properties, i.e, different factors can lead to the same effect. In addition, they are pleiotropic, meaning that one cytokine is able to mediate different effects. It has been reported about the in vivo effects of a combined administration, investigating all possible combinations of hematopoietic growth factors. Some authors showed that cytokine dose-response characteristics *in vivo* are modified in a quantitative and, in few cases, in a qualitative way by other cytokine added.
Some cytokines such as GM-CSF, SCF, IL-3, IL-6, IL-11 have been shown to stimulate granulopoiesis, however with efficiencies less than that of G-CSF, and to act synergistically with G-CSF to stimulate maximum granulopoiesis.

Among the partners of hematopoietic growth factors, in the present invention the primary attention is paid for human stem cell factor (SCF) which synergistic effect with G-CSF provides new possibilities for use of combination thereof in clinic. As it is well known, both proteins are used for the treatment of neutropenia in oncologic patients after applying of chemotherapy or in bone marrow transplantation to restore the normal number of neutrophils (granulocytes). It has been established that joint effect of treatment with both individual proteins, is significantly better in comparison with treatment with separate proteins. Synergistic effect is shown on the recombinant form of both SCF and G-CSF [J. A. Glaspy et al. Peripherial Blood Progenitor Cell Mobilization using Stem Cell Factor in Combination with Filgrastim in Breast Cancer Patients. Blood, vol. 90, pp 2939-2951, 1997/C. H. Moskowitz et al, Recombinant Metionyl Human Stem Cell Factor and Filgrastim for Peripheral Blood Progenitor Cell Mobilization and Transplantation, Blood, Vol 89, No 9, pp.3136-3147, 1997].

Patent EP0992579 (prior. 1989-10-16 etc.) discloses stem cell factor (SCF), the method of obtaining thereof, pharmaceutical composition and various methods for treatment of such conditions as leucopenia, thrombocytopenia, anemia, methods for enhancing bone marrow recovery in treatment of radiation, chemotherapy-induced bone marrow aplasia or myelosuppression. SCF is useful alone or in combination with one or more additional hematopoietic factors, such as G-CSF in the treatment of hematopoietic disorders.

It is known that recombinant form of human SCF seems capable of enhancing either filgrastim or chemotherapy in combination with filgrastim mobilizations.

EP1817047 (prior. 2004-11-05) discloses the use of granulocyte colony stimulating factor (G-CSF) polypeptide, alone or in combination with stem cell factor (SCF) polypeptide, in the prevention or treatment of brain diseases after cerebral ischemia or neurological disorder.

Initial preclinical and clinical trials have demonstrated that the transfer of bone marrow (BM)-derived stem and precursor cells into the infarcted myocardium can improve left ventricular systolic function. Similar results were obtained by the application of G-CSF alone or in combination with SCF [M. T. Kuhlmann et al. G-CSF/SCF reduces inducible arrhythmias in the infarcted heart potentially via increased connexin43 expression and arteriogenesis. JEM vol. 203, 2006 87-97].

Combination therapy by G-CSF and SCF after cerebral ischemia is effective for functional recovery, using the cytokine-induced generation of neuronal cells from both bone marrow-derived cells and intrinsic neural stem/progenitor cells. Because G-CSF and SCF are available for clinical use, these findings suggest a new therapeutic strategy for stroke treatment [H. Kanada et al. Administration of hematopoietic cytokines in the subacute phase after cerebral infarction Is effective for functional recovery facilitating proliferation of intrinsic neural stem/progenitor cells and transition of bone marrow-derived neuronal cells. Circulation 2006, 113:701-710].

SCF and G-CSF are neuroprotective and beneficial to functional restoration when administered during the acute phase after brain ischemia, indicating hematopoietic growth factors really play a role in brain repair [Li-Ru Zhao et al. Brain repair by hematopoietic growth factors in a rat model /Stroke, 2007, 38: 2584-2591; Z. E. Toth et al. The combination of granulocyte colony-stimulating factor and stem cell factor significantly increases the number of bone marrow -derived endothelial cells in brains of mice following cerebral ischemia. Blood, 2008, vol. 111, No 12].

Systemic administration of SCF together with G-CSF to transgenic mice, carrying amyloid precursor, leads to long-term reduction of β-amyloid deposition in the brain. This provides insights into the contribution of the hematopoeitic growth factors, SCF and G-CSF, to limit β-amyloid accumulation in Alzheimer disease (AD) and may offer a new therapeutic approach. for AD [B. Li et al. Stem cell factor and granulocyte colony-stimulating factor reduce β-amyloid deposits in the brains of APP/PS1 transgenic mice. Alzheimer Research & Therapy 2011, 3:8].
One of the first attempts of stem cell-based therapy using SCF and G-CSF in the treatment of renal ischemia/reperfusion was described in G. Stokman et al. Hematopoietic Stem Cell Mobilization Therapy Accelerates Recovery of Renal Function Independent of Stem Cell Contribution. J Am Soc Nephrol 16: 1684-1692, 2005.

Therefore, the clinical indications of G-CSF and its combination therapy with SCF suggested large possibilities for application of these both therapeutically important protein. However, it is worth attention that both cytokines have not been used yet in the form of single dosage form. In this respect the aim of present invention is to provide new individual substance and - on the base thereof - new "biobetter" pharmaceutical preparation in the form of heteromultimer (heterodimer) construction, containing properly connected monomer units of both proteins into one entity, seeking of :
- maximally retained biological activity of both heteromultimer (heterodimer) partners;
- extended circulation half-life *in vivo;*
- possibly gained new synergistic activity of the whole heteromultimer (heterodimer) construction as well as possible new therapeutic indications;
- lower costs using one entity instead of two separate preparations.

### Fused proteins

Some attempts to obtain heterodimer constructions of different growth factors are described in scientific and patent literature. WO9206116 (prior.1990-09-29) discloses a recombinant hematopoietic molecule comprising a first hematopoietic molecule having early myeloid differentiation activity and a second hematopoietic molecule having later myeloid differentiation activity. In a preferred embodiment of the invention, the first hematopoietic molecule (IL-3 or GM-CSF) is linked to the second hematopoietic molecule (EPO, G-CSF, IL-5 or M-CSF) by an amino acid linker sequence comprising at least two amino acid residues. In particular, IL-3-G-CSF hybrid growth factor was reported to stimulate AML193 cell proliferation to a greater extent than did the mixture of the two cytokines.

However the two cytokine activities were neither synergistic, nor were they additive. The PK/PD profile of this constructs is not provided and it is not clear whether this construct exhibit longer circulation half-life.

US 2007/081979 A1 (publ. 2007-04-12) disclosed multifunctional chimeric receptor agonists proteins, covered by the formula, selected from R1-L1-R2, R2-L1-R1, R1-R2 or R2-R1, wherein linker L1, if present, comprises the sequence (GlyₙSer)ₙ or (AlaGlySer)ₙ, and wherein individual fusion partners are comprising a modified amino acid sequence with the part of amino acid residues deleted from N- and C-terminus and/or characterized by rearrangement of native sequences of said individual fusion partners, thereby creating new C- and N-termini.

Several attempts to construct derivatives of hematopoietic cytokines fused via linker amino acid sequence to obtain dual function are also reported in non-patent publications.
However some of fusion proteins were designed unsuccessfully and their activity was lower than expected. The reason may be non proper choice of the linker sequence or non proper order of partners bonded into fusion which may cause unfavourable steric hindrance. In this context it has been shown that the modification of C-terminus part of G-CSF molecule by fusing with SCF *via* its Lys¹⁴⁸ residue has no impact neither on G-CSF biological activity nor for proliferation test [Oshima et al., Biological activity of human granulocyte colony stimulating factor with a modified C-terminus. Biochemical and Biophysical Research Communications 267, 924-927, 2000]. In opposite, the modification of N-terminus part of G-CSF by linking for example inactive diphtheria toxin leads to the decrease by 200 times the ability of the protein to stimulate formation of granulocyte colonies and thereby confirms that N-terminus part of G-CSF is important for binding with receptor.

Low *in vitro* activity of some heterodimer proteins may be caused by negative impact of fusion partners on the fusion conformation. Heterodimer GCSF-T_{f} with the longest α-helical formative linker sequence demonstrated 10 times lower in vitro cell proliferation activity due to interfering domains of heterodimer. Such interference tends to reduce binding of fused protein to G-CSF and, e.g., T_{f} receptors, thus reducing in vivo myelopoietic effect in animal trials [Y.Bai and W.-C.Shen. Improving the oral efficacy of recombinant granulocyte colony-stimulating factor and transferin fusion protein by spacer optimization [Pharmaceutical Research, Vol. 23, No.9, September 2006].

Several publications also described hybrid molecules of stem cell factor (SCF) with other cytokines, where both partners were connected via peptide linker of GGGGSGGGGSGG type by head-to-tail mode.

For above cited G-CSF hybrid constructions with other cytokines any data on the synergy of both cytokine partners and possible effects of extension residence time of hybrid construction are not provided. Prolongation of residence time was observed for G-CSF fusions with IgG or transferin, however in both cases the decrease of G-CSF activity compared to non modified G-CSF monomer form was indicated. For SCF fusions with other cytokines the increase of SCF activity is observed when comparing with monomer form thereof, however there are no indications on residence time of the fusion in serum or possible extension thereof.

Accordingly the main object of the present invention is to propose method for production of "biobetter"' version of biopharmaceutical preparations with improved properties, composed of two partners of synergistically acting hematopoietic growth factor by their genetic fusion into heterodimer, in which both protein partners are connected via linker of defined length. Sequence and structure of the linker is designed to provide ability of each monomer chain to interact with specific receptor of selected therapeutic protein and exhibit as much as possible biological and functional activity of each partner of multimer construction.

The molecular mass of heterodimer construction is increased and constituted the sum of each monomer unit molecular mass plus molecular mass of the linker. Therefore, it is another object of the present invention to provide a heterodimer construction form of therapeutic proteins of interest possessing longer *in vivo* circulation half-life than serum-residence time of each separate partner. This could be characteristic feature of biopharmaceuticals produced according to the proposed method. The increase of molecular mass of heterodimer construction itself does not necessarily leads to the extension of circulation time of the heterodimer construction with respect to any partner. It is known, for example, that the covalent G-CSF dimer formed *via* interchain -S-S- bonds has doubled molecular mass, however its circulation time in serum is the same as for G-CSF monomer.

It is a further object of the present invention to provide a heterodimer form of therapeutic proteins that can be produced *by* recombinant DNA technology without formation of undesirable dimer and oligomer products, formed by cross-linking *via* interchain disulfide bonds.

It is yet another object of this invention to provide reliable opportunity to obtain not only effect of activity prolongation *in vivo* but also synergistic activity of two different cytokines, thus extending the therapeutic possibilities (new indications of new dosage form) and in the same time reducing the costs of production due to application of single dosage form instead of two separate therapeutic proteins as it is used now.

Construction of heterodimer form of two different therapeutic proteins rise serious issues during technology development trying to purify the target construction to the quality level sufficient to meet the requirements specified for pharmaceutical substance. Main steps of process to be invented are not necessary analogous to the step used for the process designed for each individual partner of the heterodimer construction. The chromatography purification steps which are essential in creation of modern biotechnology processes can vary dramatically due to different chromatographic behavior of the whole construction of heteromultimer (in particular, heterodimer) comparing to the chromatographic behavior of each individual partner. Besides, molecular mass of the recombinant heterodimer construction is increased and this rise risk of non-productive aggregation, especially in case of high expression level of the protein, thus resulting in its accumulation in the form of insoluble inclusion bodies. Therefore, technology approaches applied for preparation of monomer protein are no more suitable for isolation and purification of heteromultimeric (in particular heterodimeric) protein construction. Here serious attention should be paid for the proper selection not only of each process stage but also for the compatibility of whole process steps assuring as high as possible biological activity of the protein partners at the end of purification cycle. So, this proves the necessity of new approaches at the preparation of heteromultimeric (in particular heterodimeric) proteins.

### Summary of the Invention

The main object of the present invention is fused proteins which are heterodimers of granulocyte colony-stimulating factor with other growth factors, of general formula (I)

X-L-GCSF (I)

wherein L is a linker sequence SGLEA-(EAAAK)ₘ-ALEA-(EAAAK)ₘ-ALEGS, wherein m=2-8, X denotes SCF, and the amino acid sequences of monomeric chain are identical by at least 95% to amino acid sequences of corresponding native proteins.

More preferably the fused protein according to present invention comprises linker sequence L, represented by SEQ ID NO: 5.

In the embodiment of present invention the amino acid sequences of monomeric chains are corresponding to those of native proteins, forming the fused protein of present invention represented by SEQ ID NO: 10.

Fused protein according to this invention is characterized by extended circulation half-life *in vivo* and synergetic activity.

A further main object of the present invention is the recombinant DNA encoding fused protein. Preference is provided to recombinant DNA, wherein the coding sequence is represented by SEQ ID NO: 15.

One another main aspect of present invention is a method for preparation of heterodimer protein of granulocyte colony-stimulating factor and stem cell factor, comprising the steps of:
a) construction of DNA encoding said target genetically fused protein and preparation of bacterial strain-producer;
b) cultivation of strain producer which cells comprise a DNA sequence, encoding the target protein, in a suitable culture medium;
c) lysis of the microorganism of producer and separation of the fraction of insoluble target proteins;
d) solubilization of the fraction of insoluble proteins;
e) renaturation of the target protein;
f) purification of the target protein.
Method according to present invention characterized in that: target protein is genetically fused protein of present invention;
- strain-producer in step b) is cultivated under conditions, suitable for expression of the heterodimer construction of genetically fused protein of present invention;
- fraction of insoluble proteins in step d) is solubilized in a buffer solution containing urea as chaotropic agent and dithiothreitol as reducing agent,
- renaturation of heterodimer in step e) is oxidative renaturation in a buffer solution containing intermediate urea concentration in the presence of dithiothreitol (DTT) as reducing agent and oxidized glutathione (GSSG) as oxidizing agent;
- renaturated target protein in step f) is purified by sequential anion-exchange chromatography with a further mixed-mode chromatography and cation-exchange chromatography.

Renaturation of said heterodimer protein is performed in a buffer solution of pH 7.5 - 8.5 containing 2M urea in the presence of dithiothreitol and oxidized glutathione in molar ratio DTT:GSSG of 5-1:1-5; more preferably in a buffer solution at pH 8.0 and DTT:GSSG molar ratio 1:5.

In one aspect of this invention in step f) said anion-exchange chromatography is performed on DEAE-Sepharose FF sorbent at pH 7.0 - 8.5, preferably at pH 7.5, applying step-wise elution mode; said mixed-mode chromatography is performed on CHT hydroxyapatite sorbent at pH 6.5 - 7.5, preferably at pH 7.2; said cation-exchange chromatography is performed on SP-Sepharose FF sorbent at pH 4.5 - 5.2, preferably at pH 4.7; protein solution obtained is concentrated and stored in acetic acid/NaOH buffer solution.

In the best mode of present invention in step a) said DNA, encoding target genetically fused protein, is inserted into expression plasmid pET21b and selected plasmids are transformed into bacterial *E*. *coli BL21(DE3)* strain-producer, and in step b) said strain-producer is cultivated in LB medium at 37°C up to optical density (OD₆₀₀ₙₘ) 0,8-1,2, by using isopropyl-β-D-thiogalaktopyranoside (IPTG) for induction.

Yet another preferred embodiment is fused SCF-GCSF protein, obtained by the method of present invention. The SCF-GCSF protein of present invention is intended for use in therapy.

### Brief Description of the Drawings:

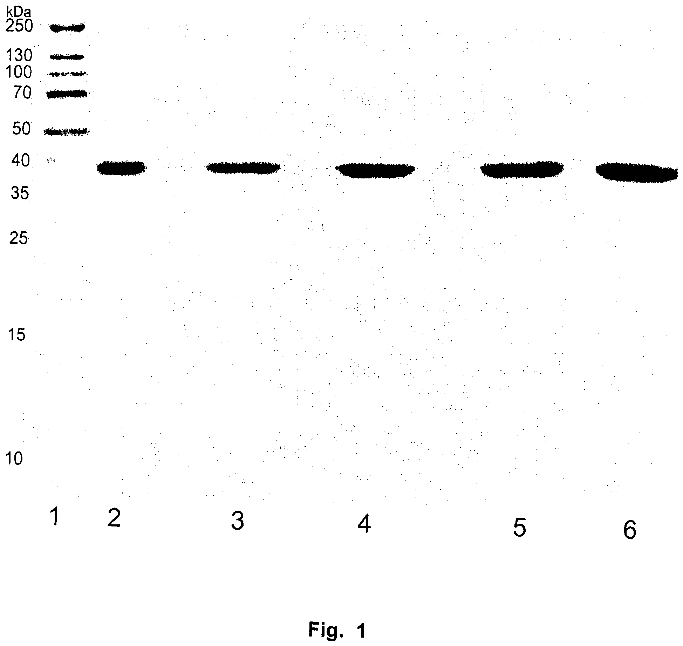

Fig. 1: presented the alteration of electrophoretic purity of SCF-G-CSF heterodimer of present invention over process stages, namely, the electrophoretic pattern of SCF-G-CSF heterodimer protein samples under reducing conditions. Lines: 1 - PageRuler™ Plus Prestained Protein Ladder #26619 - markers of protein molecular mass; 2 - sample of inclusion bodies; 3 - sample after protein renaturation; 4 - sample after protein chromatography over DEAE-Sepharose FF column; 5 - sample after protein chromatography over hydroxyapatite CHT column; 6 - sample after protein chromatography over SP-Sepharose column.
Fig. 2: Western blot analysis of purified SCF-G-CSF heterodimer protein. (A) - sample of 3 µg of SCF-G-CSF heterodimer loaded under reducing conditions; blot staining with monoclonal antibodies against G-CSF; (B) - sample of 3 µg of SCF-G-CSF heterodimer loaded under reducing conditions; blot staining with polyclonal antibodies against SCF.
Fig. 3: Purified SCF-G-CSF heterodimer protein analysis by SE-HPLC method.
   Optical density of eluate at 280 nm. Peak 1: retention time 2,329 min; peak height 2,90963 e⁻¹ mAU; area percent 2,0264%. Peak 2: retention time 14,749 min; peak height 7,56982 mAU; area percent 97,9736%.
Fig.4. Thermal stability of SCF-G-CSF heterodimer, namely, curves of SCF-G-CSF heterodimer thermal denaturation. Top curve - SCF-G-CSF heterodimer formulated in acetate buffer; bottom curve - SCF-G-CSF heterodimer formulated in PBS buffer.
Fig.5. Stimulation of absolute neutrophil count (ANC) *in vivo* in rat blood by G-CSF, SCF, mixture thereof and SCF-G-CSF heterodimer. ANC alteration in rat blood after injection of protein samples. Injected were samples of SCF-G-CSF heterodimer, SCF monomer, G-CSF monomer, and sample of SCF and G-CSF monomer mixture. Amount of neutrophils in blood was determined just after injection, then after 24hours and 48 hours after injection.
Fig. 6. Change of G-CSF concentration in rat serum over time after injection of G-CSF, SCF, SCF+G-CSF mixture and SCF-G-CSF heterodimer. G-CSF concentration in rat sera was determined 3, 6 and 18 hours after injection.

### Detailed Description of the Invention

The present invention disclosed new type of heterodimers, comprising of monomer of granulocyte colony-stimulating factor genetically fused *via* linker sequence of defined length with monomer of other hematopoietic growth factor.

Recombinant form of proposed according to present invention heterodimers is formed of genetically fused recombinant biologically active and synergistically acting protein monomer chains, connected via suitable linker, wherein both protein monomer chains have amino acid sequences of native biologically active protein or are essentially the same sequences of at least 95% identity. The suitable linker moiety has an amino acid sequence SGLEA-(EAAAK)ₘ-ALEA-(EAAAK)ₘ₋ALEGS, wherein m=2-8. In preferred embodiment of present invention said suitable linker moiety has an amino acid sequence, represented by SEQ ID No:5 (Table 1).

**Table 1 Examples of used linker sequences (linker sequences SEQ ID NO:1 to SEQ ID NO:4 are not part of the invention)**

| Sequence Number | Identification | Linker sequence | Number of amino acids |
|---|---|---|---|
| SEQ ID No:1 | L2 | (SG₄)-(SG₄)-S | 11 |
| SEQ ID No:2 | L3 | (SG₄)-(SG₄)-(SG₄)-S | 16 |
| SEQ ID No:3 | L5 | (SG₄)-(SG₄)-(SG₄)-(SG₄)-(SG₄)-S | 26 |
| SEQ ID No:4 | L7 | (SG₄)-(SG₄)-(SG₄)-(SG₄)-(SG₄)-(SG₄)-(SG₄)-S | 36 |
| SEQ ID No:5 | Lα | SGLEA-(EAAAK)₄-ALEA-(EAAAK)₄-ALEGS | 54 |

Heterodimer constructions of present invention may comprise G-CSF and other hematopoietic factor (SCF) acting synergetically with G-CSF.

One of the most preferable embodiment of present invention is heterodimer of granulocyte colony-stimulating factor and stem cell factor having amino acid sequence represented by SEQ ID No:10 (Table 2).

**Table 2 Amino acid sequences and molecular mass of heterodimer proteins (SEQ ID NO:6 to SEQ ID NO:9 are not part of the invention)**

| Sequence Number. | Heterodimer protein | Molecular mass, kDa | Amino acid sequence of heterodimer protein |
|---|---|---|---|
| SEQ ID No:6 | SCF-L2-GCSF | 38,03 | |
| SEQ ID No:7 | SCF-L3-GCSF | 38,35 | |
| SEQ ID NO:8 | SCF-L5-GCSF | 38,98 | |
| SEQ ID NO:9 | SCF-L7-GCSF | 39,61 | |
| SEQ ID NO:10 | SCF-Lα-GCSF | 42,38 | |

Another important object of the present invention is method of preparation of fused G-CSF and SCF protein as defined above and disclosed in more details in a below presented examples, which method enabled to isolate and purify the target fused protein assuring maximal possible activity of both synergetically acting therapeutic proteins.

In the structure of heterodimer according this invention full amino acid sequence of stem cell factor monomer has been selected as the N-terminal partner while as C-terminal partner is the full amino acid sequence of monomer of granulocyte colony-stimulating factor. Such an order of partners fusing into heterodimer construction *via* properly selected linker sequence appeared rationale since about all sequence of SCF (1-148) is important for its biological function. N-terminus of G-CSF is responsible for interaction with receptor.

Considering specificity of biological interactions with receptors it is important to purposefully select the order of partners genetic fusion, i.e., defining which partner will occupy N- and which C-terminus position.

According to this invention the main steps for SCF-G-CSF heterodimer protein preparation and quality control involve following stages:
- preparation of genetic construct and construction of bacterial strain-producer,
- development processes of protein biosynthesis, renaturation and chromatography purification,
- development of in-process control methods to handle protein quality,
- formulation of purified protein for long-term storage ,
- protein characterization and *in vitro* bioassay with G-CSF proliferation test,
- determination of protein pharmacokinetic and pharmacodynamic properties *in vivo.*
The genes of SCF and G-CSF heterodimer form were constructed fusing gene copies *via* linker sequence into one DNA fragment which was cloned into expression plasmid (pET-21b) and into strain-producer (*E.coli* BL21(DE3). Selected clones were cultivated in a proper selected medium (LB medium) at +37°C. Biosynthesis of the target protein was induced by IPTG (isopropyl-β-D-thiogalactopyranoside). Lysate of biomass was prepared by boiling with sodium dodecylsulfate (SDS), and was analyzed by electrophoresis. Prepared constructions of fused G-CSF proteins are specified in Table 2.
Sequences of heterodimer constructions were controlled and verified by determining primary nucleotide sequence which corresponded to predicted heterodimer sequence encoding both protein partners connected *via* linker sequence. Linker amino acids sequence containing element of alpha helix structure EAAAK with periodic repeats of this structure from 2 to 8 times was selected.
It was found that the use of 54 amino acids residue sequence which is in the form of structurised alpha helix it is possible to distance both partners of heterodimer construction in such a length which exclude their inter-interaction and steric hindrance assuring free interaction of each partner with its specific receptor. Such construct of heterodimer allow both partners exhibit the synergy of their biological function by a similar mode as their combined use in solution. In addition to this the developed heterodimer construction exhibited *in vivo* longer survival time in comparison to that of monomeric form of SCF or G-CSF.

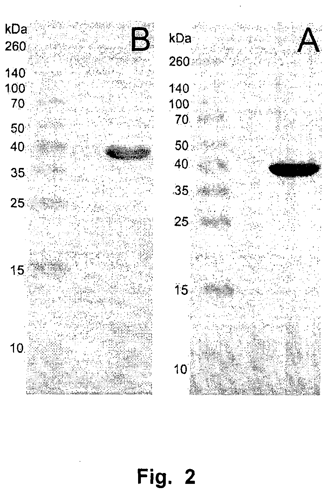

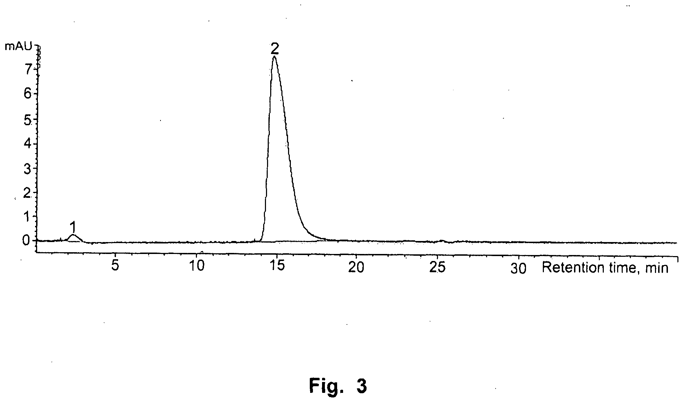

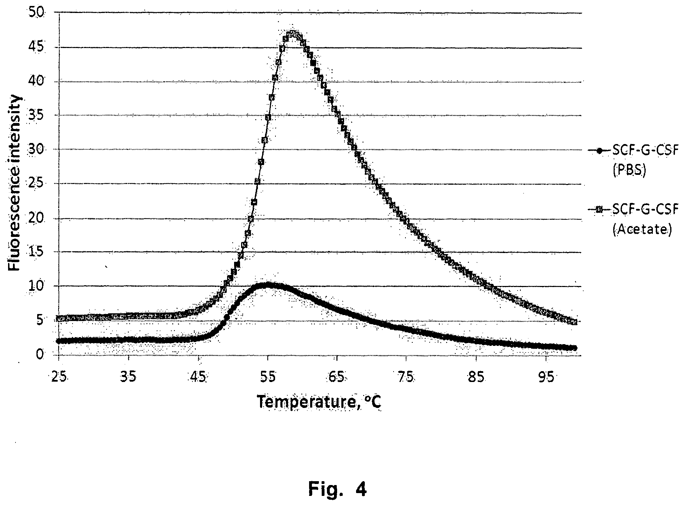

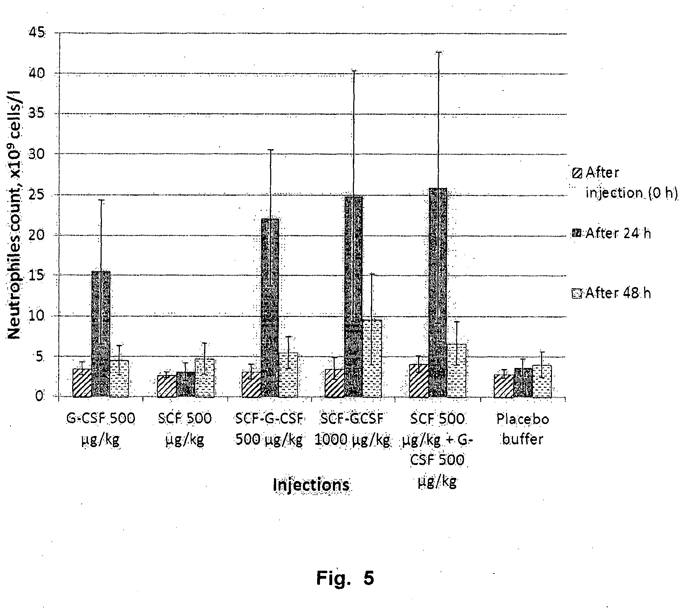

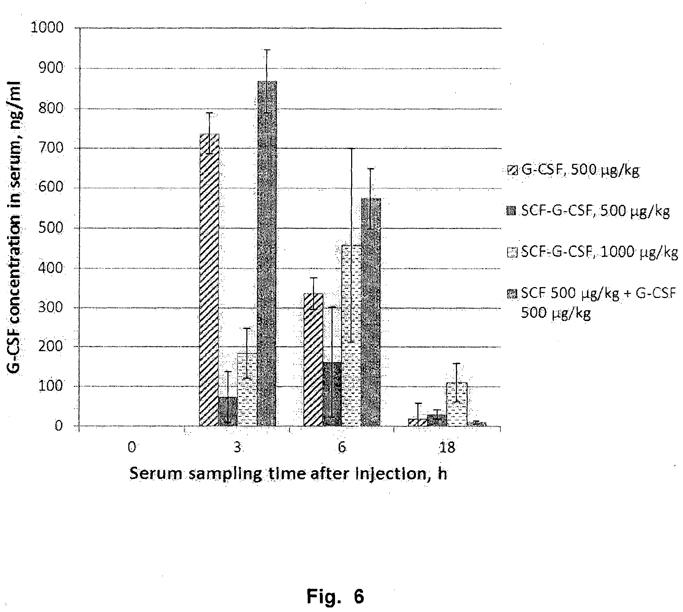

Conformance of amino acids sequence of heterodimer to theoretical gene encoding sequence was confirmed by mass spectrometry (MS) analysis of purified protein checking the conformity of determined molecular mass to theoretical. Functionality of both heterodimer partners was confirmed by Western blot analysis (Fig 2) using antibodies specific to SCF and G-CSF parts of heterodimer. Purity of heterodimer and purity alteration over protein purification steps is presented in Fig. 1. In addition to this, the association status of purified heterodimer was determined by analytical SE-HPLC and high 98% monomer content of fused SCF-G-CSF heterodimer protein was found (Fig. 3). Finally purified protein substance was formulated in a two distinct buffer systems and thermal protein denaturation patterns were determined. This allowed choose acetate buffer system (Fig. 4), as appropriate for long-term storage of the protein and provide formulated protein for *in vivo* studies. In vitro bioassay of heterodimer G-CSF part in proliferation test using mouse myeloid leukaemia cell line G-NFS-60 (13 example) and alteration of neutrophil count in rat model system (Fig. 5) confirmed the correctness of heterodimer structure and its biological activity. Apart that, the alteration of G-CSF concentration in rat blood over time revealed the extension of heterodimer circulation time (Fig. 6).
The substance of purified protein is to be used for formulation of pharmaceutical composition. The heterodimer of granulocyte colony-stimulating factor and stem cell factor is designated to produce active pharmaceutical substance and pharmaceutical composition and dosage form on its basis for treating of diseases and conditions in same indications where monomeric form of granulocyte colony-stimulating factor and stem cell factors protein or combination of both is currently used, including oncohematology, treatment of neurological diseases and ophtalmology.
On the basis of this invention the pharmaceutical composition, comprising a therapeutically effective amount of heterodimer of granulocyte colony-stimulating factor and stem cell factor as above might be easily compiled in combination with pharmaceutically acceptable carrier, diluent, excipient and/or auxiliary substances.

Besides pharmaceutically effective amount of fused protein defined above, such pharmaceutical composition could be formulated with following further ingredients:
- carier compounds such as monosaccharide (glucose, dextrose), disaccharides (sacharose, fructose) polyhydroxyl alcohols (sorbitol, mannitol);
- buffer substance for defined pH value (acetic, phosphorous, citric acids, Tris substance, Good buffer substances);
- additive of salts for isotonicity (NaCl, sodium or ammonium sulphate);
- surface active agents (detergents) for protection of protein from damage and degradation at the air-liquid interface (polysorbate 20 or 80, Pluronic type compounds);
- stabilizing agents (polyethylene(propylene) glycols, amino acids Met, His, Asp, Arg, Gly);
- chelating agents (EDTA, EGTA, IDA);
- antimicrobial agents (cresol, benzyl alcohol);
- SH-agents (glutathione, Cys, acetylCys);
- other auxiliary compounds.

### Embodiments of the Invention

Represented below is information on specific examples on preparation of fused G-CSF proteins, and properties thereof. These examples are presented for illustrative purpose and are not limiting the scope of the present invention.

### Examples 1-5

### Construction of SCF-G-CSF heterodimer protein expression vector and strain-producer

DNA sequences for target fusion protein construction were obtained using synthetic DNA fragments. Human SCF gene fragment was constructed with the restriction sites for the enzymes NdeI and Kpn2I on the fragment 5' and 3' ends respectively. Linker sequences of L2, L3, L5, L7 (where L2-L7 are not part of the invention) Lα fragments were obtained with the restriction sites of Kpn2I and BamHI on the fragment 5' and 3' ends respectively. Human G-CSF gene fragment was obtained with the restriction sites BamHI and HindIII on the fragments 5' and 3' ends respectively (coding sequence of STOP codon was included into the 3'-end of the fragment). Each sequence in the Table 3 below was obtained by combining SCF fragment with G-CSF fragment joined via selected linker sequence with the help of DNA ligase. The fragments obtained were exposed with restriction enzymes Ndel and Hindlll, and ligated into an expression plasmid pET21b (Novagen) at the same sites. Final recombinant plasmid was sequenced. Constructed variants of the sequences are given in the Table 3. Selected plasmids were transformed into the bacteria strain *E*. *coli BL21 (DE3)*. The selected colonies were cultivated in LB medium at 37°C to the optical density (OD₆₀₀ₙₘ) 0.8-1.2. Biosynthesis of heterodimeric protein was induced with 0.5-1 mM IPTG (isopropyl- β -D- thiogalactopyranoside), induction time was 2.5-3 h. Biomass lysates obtained by the boiling in 1% of sodium dodecylsulfate (SDS) were analyzed by the method of polyacrylamide gel electrophoresis.

**Table 3 Gene sequences for heterodimer proteins (SEQ ID NO:11 to SEQ ID NO:14 are not part of the invention)**

| Examp le No. | Number of DNA sequence and fused protein | Sequence of fused protein |
|---|---|---|
| 1 | SEQ ID NO: 11 SCF-L2-GCSF | |
| 2 | SEQ ID NO: 12 SCF-L3-GCSF | |
| | | |
| 3 | SEQ ID NO:13 SCF-L5-GCSF | |
| 4 | SEQ ID NO:14 SCF-L7-GCSF | |
| | | |
| 5 | SEQ ID NO: 15 SCF-Lα-GCSF | |

Appropriate are various versions of DNA sequence that encodes the sequences of native partners of heterodimer construction or sequence identical to native by at least 95%.

In below cited examples SCF-GCSF heterodimer means construct of SCF and G-CSF monomer units fused via linker sequence of Lα protein according the invention, which amino acids sequence is SEQ ID NO: 10 (Table 2). SEQ ID NO:6 to SEQ ID NO:9 are provided to compare the properties.

### Example 6

### Biosynthesis of SCF-G-CSF heterodimer protein

SCF-G-CSF heterodimer producer *E.coli* BL21 (DE3) pET21b-SCF-GCSF-Lα was grown in flasks and/or bioreactor.

For inoculum preparation 60 ml LB medium (*MO BIO laboratories, Inc*.) in 250 ml flask was used. Ampicillin (*Roth*) concentration was 100 mg/l. Flasks were shaked at +37°C with 220 rpm agitation. After ∼16 hours the culture was transferred into 5L volume bioreactor (*Sartorius AG*) with effective medium volume of 3L (2% v/v of inoculum). The medium consisted of 11.28 g/L M9 type media (Sigma); and 5 g/L yeast extract (*Fluka*). The additives of media as 4 g/L glucose, 1 g/L MgSO₄ (*Sigma*); 100 mg/L ampicillin were sterilized separately and added to bioreactor aseptically. Fermentation was run at +37°C; keeping medium pH at 6.8 automatically adjusting with 1M solutions of NaOH or 1M HCI; and keeping saturation by O₂ at -20% with the aid of air velocity and agitation speed changes. After the optical density at 600 nm (OD600) reached value 1.3 - 1.6, the IPTG (isopropyl-β-D-thio-galactopyranoside) was added for induction (final concentration 1 mM) and fermentation was continued for further 2 hours. Biomass was collected by centrifugation at +4°C for 30 min at 3500 - 4000 rpm. Biomass was stored in frozen state at -20°C.

The expression level of target SCF-G-CSF heterodimer protein constitutes 20.95 ± 3.45 %.

### Example 7

### Isolation of SCF-G-CSF heterodimer inclusion bodies (IB) from E.coli cells

12 g of frozen *E.coli* biomass was homogenized in 240 ml of 0.1 M Tris-HCl buffer pH 7.0 containing 5 mM EDTA adding further 0.24 g lysozyme, 0.24 ml Triton X-100, 2.4 ml of 100 mM phenylmethylsulfonylfluoride (PMSF) solution in ethanol and 1.68 ml 2-mercaptoethanol (final concentration 100 mM). Homogenizate is stirred on magnetic stirrer for 30 min at room temperature, and the cells were disrupted by ultrasonic (22kHz) for 10 minutes in ice bath and centrifuged with *Beckman* centrifuge at +4°C for 25 minutes at 14000 rpm. The pellet of IB was collected and washed two times by homogenization in 240 ml solution of 20 mM Tris-HCl buffer pH 8.0 containing the additives of 1 M NaCl and 0.1% polysorbate 80 and once with 240 ml of 20 mM Tris-HCl buffer pH 8.0. After each homogenization cycle the suspension obtained was centrifuged with *Beckman* centrifuge at +4°C for 25 minutes at 14000 rpm.

### Example 8

### Solubilization of SCF-G-CSF heterodimer inclusion bodies in the presence of reducing agent

IB isolated from 12 g of *E.coli* biomass were solubilized in 120 ml of 50 mM Tris-HCI buffer pH 8.0 containing 8 M urea, and 0.5 mM DTT with mixing on magnetic stirrer for 2 hours at +4°C After that the solubilisate was centrifuged with *Beckman* centrifuge at +4°C for 25 minutes at 14000 rpm.

### Example 9

### Renaturation of SCF-G-CSF heterodimer

Solubilisate of IB in a buffer solution containing 8M urea (185 mg of total protein) was diluted 4 times with 50 mM Tris-HCl buffer pH 8.0. Heterodimer protein was renatured in 50 mM Tris-HCl buffer pH 8.0 containing 2M urea, 0.125 mM DTT, and 0.625 mM oxidized glutathione (GSSG) by gentle mixing for 24 hours at +4°C. After that the renaturation solution was centrifuged with *Beckman* centrifuge for 25 minutes at 14000 rpm.

### Example 10

### Anion-exchange chromatography of SCF-G-CSF heterodimer protein over DEAE-Sepharose FF sorbent

Purification of renatured SCF-G-CSF heterodimer protein is performed at room temperature by using the column containing DEAE-Sepharose FF anion-exchanger and ÄKTA *Purifier* chromatography system.

Glass column (XK 26 or XK 16/20, "GE Healthcare") containing 60 ml of DEAE-Sepharose FF sorbent was equilibrated with 50 mM Tris-HCI buffer pH 7.5. Solution of renatured SCF-G-CSF heterodimer (185 mg protein containing 27% of correctly folded heterodimer protein determined by RP-HPLC analysis) was loaded into the column at flow-rate of 5 ml/min. Not retained protein was removed by column washing with 5 column volumes (CV) of 50 mM Tris-HCI buffer pH 7.5 at a flow-rate of 3 ml/min. Absorbance of eluate fractions (volume of 15 ml each) was monitored at 280, 254 and 215 nm. Adsorbed protein was eluted from the column by step-wise elution mode increasing the ionic strength of eluent by steadily increasing the concentration of NaCl (Table 4) from 0 M to 0.5 M over total 8 CV. Eluent flow-rate was 3 ml/min.

**Table 4 Step-wise elution over DEAE-Sepharose FF column**

| Step number | Concentration of eluent | Number of column volumes (CV) |
|---|---|---|
| 1 | 20% (0,1 M NaCl) | 1 |
| 2 | 30% (0,15 M NaCl) | 1 |
| 3 | 40% (0,2 M NaCl) | 1 |
| 4 | 50% (0,25 M NaCl) | 1 |
| 5 | 100% (0,5 M NaCl) | 4 |

Samples from eluate fraction (10 ml each) were drawn for protein analysis by Bradford assay, for target protein amount per cent by RP-HPLC analysis and purity assessment by SDS-PAGE. Fractions containing highest amount of target correctly folded heterodimer protein were combined for further chromatography step.

After chromatography cycle the sorbent was regenerated.

### Example 11

### Mixed-mode chromatography of SCF-G-CSF heterodimer protein over hydroxyapatite CHT sorbent

The pool of combined fractions with heterodimer protein recovered from DEAE-Sepharose FF column was further chromatographied over hydroxyapatite CHT (CHT™ Ceramic Hydroxyapatite) sorbent. Glass column (XK 16/20, "GE Healthcare") containing 18 ml of CHT hydroxyapatite was connected to ÄKTA *Purifier* chromatography system and equilibrated at room temperature with 50 mM Tris-HCI buffer pH 7.2. SCF-G-CSF heterodimer protein recovered from DEAE-Sepharose FF column (18 mg total protein with 63% of correctly folded heterodimer) was loaded into the column at a flow-rate 3 ml/min. Not retained protein was removed by washing with 5 CV of 5 mM NaH₂PO₄ buffer pH 7.2 containing 0.1 M NaCl at a flow-rate of 3 ml/min. Absorbance of eluate fractions (10 ml volume, each) was monitored at 280, 254 and 215 nm. Adsorbed protein was eluted from the column with linear gradient of NaH₂PO₄ concentration from 5 mM to 500 mM over 10 column volumes (CV) at a flow-rate of 3 ml/min. Samples from eluate fraction (5 ml each) were drawn for protein analysis by Bradford assay, analysis of target protein per cent by RP-HPLC and purity assessment by SDS-PAGE. Fractions containing highest amount of target correctly folded heterodimer protein were combined for further chromatography step.

The rest of adsorbed proteins were removed from the column by washing with 5 CV of 0.5 M NaH₂PO₄ solution pH 7.0.

### Example 12

### Cation-exchange chromatography of SCF-G-CSF heterodimer protein over SP-Sepharose FF sorbent

The third SCF-G-CSF heterodimer chromatography purification step is performed over SP-Sepharose FF cation-exchanger. For this purpose, the glass column (XK 16/20, "GE Healthcare") containing 8 ml of sorbent was connected to ÄKTA *Purifier* chromatography system and equilibrated at room temperature with 20 mM Na acetate buffer pH 4.7. Protein solution recovered from hydroxyapatite CHT column (5 mg protein containing 77% of correctly folded heterodimer as judged by RP-HPLC analysis) was loaded onto the column at a flow-rate of 3 ml/min. Not retained protein was washed out with 5 CV of 20 mM Na acetate buffer pH 4.7 at a flow-rate of 3 ml/min.

Absorbance of eluate fractions (5 ml volume, each) was monitored at 280, 254 and 215 nm. Adsorbed protein was eluted from the column with linear gradient of NaCl from 0 mM to 500 mM over 10 column volumes (CV) at a flow-rate of 3 ml/min. Samples from eluate fraction (3 ml each) were drawn for protein analysis by Bradford assay, analysis of target protein per cent by RP-HPLC and purity assessment by SDS-PAGE. Fractions containing highest amount of target correctly folded heterodimer protein were combined and concentrated to about 1 mg/ml and transferred to storage buffer system e.g., 20 mM acetic acid/NaOH buffer pH 4.0 by diafiltration using Amicon® Ultra-15 filter devices. After that the protein solution is filtered over 0.22 µm *MustangE* type filter device retaining endotoxins. Purified protein solution in Na acetate buffer is stored at +4°C.

### Example 13

### Testing of SCF-G-CSF heterodimer in vitro biological activity

*In vitro* biological activity of SCF-G-CSF heterodimer was determined using G-NFS-60 cell line in RPMI 1640 growth medium [Weinstein Y, Ihle JN, Lavu S, Reddy EP, Truncation of the *c-myb* gene by retroviral integration in an interleukin 3-dependent myeloid leukemia cell line. Proc. Natl. Acad. Sci. USA, 1986, vol. 83, p. 5010-5014]. Biologically active G-CSF protein stimulated proliferation of this cell line upon G-CSF interaction with its receptor on the surface of these cells. For bioassay the G-NFS-60 cells are cultivated in 96 wells plate for 48-72 hours adding into growth medium various amounts (0,1-10 ng/ml) of tested heterodimer sample. As a reference, the sample of Neupogen™ (active substance Filgrastim), which claimed specific biological activity is 100 MIU/mg) is used. Cell proliferation is evaluated by colorimetry using MTS dye. The number of viable cells is directly proportional to biological activity of G-CSF. This evidenced, that the heterodimer may interact *in vivo* with respective receptors eliciting biological events, which might be exploited for therapeutic purposes. The determined biological activity of SCF-G-CSF heterodimer is equal to 14,95±3,88 MIU/mg.

### Example 14

### Testing of SCF-G-CSF heterodimer biological function in vivo in rats modeling systems

Pharmacokinetic (pK) and pharmacodynamic (pD) parameters of SCF-G-CSF heterodimer protein were evaluated *in vivo* using *Wistar* clone female 4 months age rats of 200 - 220g weight. For evaluation of proteins pK parameters the experimental rats were divided into groups of 3-5 rats in each. Testing and control protein sample were injected subcutaneously. For testing of heterodimer protein the samples containing 500 and 1000 µg of the protein per 1kg of rat weight was used. For control samples the G-CSF preparation Tevagrastim™ and *E*.*coli*-derived recombinant human SCF prepared by internal Profarma UAB technology and the mixture of these both proteins with the same protein concentrations referring to 1kg of animal weight were used. For concentration testing of recombinant protein the blood samples were collected after 3, 6 and 18 hours post injection. Blood samples of 0.8 - 1.0 ml were taken from rat tail vein, incubated at room temperature for 1.0 - 1.5 hours and for 12 hours at fridge at + 4°C. For serum separation the samples were centrifuged at room temperature for 15 min at 1500 rpm and the collected serum was aliquoted into 2-3 sterile tubes and frozen at -80°C.
Before the start of experiments and 24, 48 and 72 hours post injection the blood samples were analyzed with blood analyzer *Hemavet 950.* Pharmacodynamic effect of tested recombinant proteins was observed by analysis of the dynamic of blood neutrophils (granulocytes) count alteration. For this purpose samples of 20 µl blood from rat tail vein were drawn, mixed with 5 µl of 7.5% solution of EDTA, incubated for 10 min and analyzed with *Hemavet 950.* Analysis data are presented in Fig. 5.
Samples of blood sera were tested by ELISA method with *PeproTech* kits for human G-CSF analysis in serum (Human G-CSF ELISA Development Kit). Analysis data are presented in Fig.6.

Data presented in Fig. 5 showed that neutrophils count after injection of SCF-G-CSF heterodimer protein was higher than the count after injection of G-CSF monomer at its 500 µg/kg and 1000 µg/kg doses. In the case of SCF-G-CSF heterodimer, neutrophils count value after 48 hours was higher than in the case of SCF and G-CSF monomer mixture, and enabled to state that SCF-G-CSF heterodimer protein functioned longer than protein monomer mixture. From the other part the mixture of SCF and G-CSF monomer exhibited synergy of their function compared to the function of separate protein. Analogous synergy exhibited heterodimer form of SCF-G-CSF confirming the fact that properly designed structure of heterodimer may save the synergy effect of partner functioning. This evidenced the advantages for the use of such a fused protein constructions for therapy as hematopoietic agent in oncohematology, as neurotropic agent for the treatment of neurological diseases and as neuroprotective agent in ophthalmology. Besides there is no needs to use registered forms of both protein drug products - it is more rational to develop adequately acting heterodimer constructions.
Data presented in Fig. 6 showed that the survival profile of heterodimer protein over time is different from the alteration over time of G-CSF monomer form, which concentration after 6 hours decrease by more than 2 times, while in the case of heterodimer its concentration increased over the same time by more than twice. Differently from G-CSF monomer, the SCF-G-CSF heterodimer form remains in the serum and after 18 hours, indicating that its residence time in serum is longer by about three times than in the case of G-CSF monomer (Tevagrastim™).

Therefore, heterodimer protein according to this invention exhibited extension of circulation time in serum. This indicated that at the proper design of two selected protein fusion it is possible to achieve the longer circulation time in serum of the heterodimer form which *in vivo* is able to play the same role as the mixture of both nonmodified protein monomers.

In the present invention the extension of circulation half-life is achieved by increasing the molecular mass of heterodimer construction as well as properly selecting the order of partners fusion and the structure and the length of linker sequence.
Both structure of developed heterodimer construction and selection of heterodimer isolation and purification process steps along with compatibility thereof within the whole process confirmed that the equivalent amount of heterodimer will exhibit *in vivo* synergetic effects of biological function, similarly as combined functioning of both partners.

### Industrial applicability

This invention has large implementation options for therapeutic use.

## Claims

1. A fused protein, which is heterodimer of granulocyte colony-stimulating factor with other growth factor, of general formula (I)
X-L-GCSF (I)
wherein L is the linker sequence SGLEA-(EAAAK)ₘ -ALEA-(EAAAK)ₘ -ALEGS, wherein m=2-8, X denotes SCF, and the amino acid sequences of monomeric chains are identical by at least 95% to amino acids sequences of corresponding native proteins.

2. The fused protein according to claim 1, wherein linker sequence L is represented by SEQ ID NO: 5.

3. The fused protein according to any one of claims 1-2, wherein the amino acid sequences of monomeric chains are corresponding to those of native proteins, forming fused protein represented by SEQ ID NO:10.

4. The fused protein according to any one of preceding claims, **characterized by** extended circulation half-life *in vivo* and synergetic activity.

5. A recombinant DNA encoding fused protein according to claims 1-4.

6. Recombinant DNA according to claim 5, wherein coding sequence is represented by SEQ ID NO: 15.

7. Method for preparation of heterodimer protein of granulocyte colony-stimulating factor and stem cell factor according to any of claims 1-4, comprising the following steps:
a) construction of DNA, encoding target genetically fused protein and preparation of bacterial strain-producer;
b) cultivation of strain-producer, which cells comprise DNA sequence, encoding target protein in a suitable culture medium;
c) lysis of the microorganism of producer and separation of the fraction of insoluble target proteins;
d) solubilization of the fraction of insoluble proteins;
e) renaturation of the target protein;
f) purification of the target protein,
**characterised in that**: target protein is genetically fused protein according to claims 1-4;
- strain-producer in step b) is cultivated under conditions, suitable for expression of the heterodimer construction of said genetically fused protein;
- fraction of insoluble proteins in step d) is solubilized in a buffer solution, containing urea as chaotropic agent and dithiothreitol as reducing agent,
- renaturation of heterodimer in step e) is oxidative renaturation in a buffer solution, containing intermediate urea concentration in the presence of dithiothreitol (DTT) as reducing agent and oxidized glutathione (GSSG) as oxidizing agent;
- renaturated target protein in step f) is purified by sequential anion-exchange chromatography with a further mixed-mode chromatography and cation-exchange chromatography.

8. The method according to claim 7, **characterised in that** said renaturation of heterodimer protein is performed in a buffer solution of pH 7.5 - 8.5 containing 2M urea in the presence of dithiothreitol and oxidized glutathione in molar ratio DTT:GSSG 5-1:1-5; preferably in a buffer solution at pH 8.0 and DTT:GSSG molar ratio 1:5.

9. The method according to claim 8, **characterised in that** in step f) said anion-exchange chromatography is performed on DEAE-Sepharose FF sorbent at pH 7.0 - 8.5, preferably at pH 7.5, applying stepwise elution mode; said mixed-mode chromatography is performed on CHT hydroxyapatite sorbent at pH 6.5 - 7.5, preferably at pH 7.2; said cation-exchange chromatography is performed on SP-Sepharose FF sorbent at pH 4.5 - 5.2, preferably at pH 4.7; obtained protein solution is concentrated and stored in acetic acid/NaOH buffer solution.

10. The method according to claim 9, **characterised in that** in step a) said DNA, encoding target genetically fused protein according to claims 1-4, is inserted into expression plasmid pET21b and selected plasmids are transformed into bacterial *E. coli BL21(DE3)* strain-producer, and in step b) said producer is cultivated in LB medium at 37°C temperature up to optical density (OD₆₀₀ₙₘ) 0,8-1,2, using isopropyl-β-D-thiogalactopyranoside (IPTG) as inductor.

11. Fused SCF-GCSF protein, obtained by the method of claim 10 for use in therapy.

## Patentansprüche

1. Fusionprotein nach der allgemeinen Formel (I), welches Heterodimer eines Granulozyten-Kolonie-stimulierenden Faktors und eines anderen Wachstumsfaktors ist
X-L-GCSF (I)
wobei L die Verbindungssequenz SGLEA-(EAAAK)ₘ -ALEA-(EAAAK)ₘ ist, worin m=2-8, X bezeichnet SCF, und die Aminosäuresequenzen von monomeren Ketten mindestens 95% identisch mit Aminosäuresequenzen von entsprechenden nativen Proteinen seien.

2. Fusionprotein nach Anspruch 1, wobei die Verbindungssequenz L dargestellt wird durch SEQ ID NO: 5.

3. Fusionprotein nach einem der Ansprüche 1-2, wobei die Aminosäuresequenzen monomerer Ketten denen von nativen Proteinen entsprechen, bildet ein verkettetes Protein, dargestellt durch SEQ ID NO: 10.

4. Fusionprotein nach einem der vorhergehenden Ansprüche ist charakterisiert durch eine verlängerte Kreislauf-Halbwertszeit *in vivo* und synergetische Aktivitäten.

5. Eine rekombinante DNA, die für ein Fusionsprotein gemäß den Ansprüchen 1-4 codiert.

6. Die rekombinante DNA nach Anspruch 5, wobei die Kodierungssequenz dargestellt wird durch SEQ ID NO: 15.

7. Verfahren zur Herstellung von einem Heterodimer-Protein eines Granulozyten-Koloniestimulierenden Faktors und des Stammzellenfaktors nach einem der Ansprüche 1-4, umfassend die folgenden Schritte:
a) aufbauen der DNA, codieren des genetisch verketteten Zielproteins und vorbereiten des Bakterienstamm-Erzeugers;
b) kultivieren des Stamm-Erzeugers, dessen Zellen die DNA-Sequenz beeinträchtigen, codieren des Zielproteins in einem passendes Nährmedium;
c) Lyse des Mikroorganismus des Erzeugers und Trennung von dem Anteil nicht-löslicher Zielproteine;
d) Solubilisierung des Anteils nicht-löslicher Proteine;
e) Renaturierung des Zielproteins;
f) Aufreinigung des Zielproteins,
die sich dadurch auszeichnet, dass das Zielprotein ein genetisch verkettetes Protein nach den Ansprüche 1-4 ist;
- der Stamm-Erzeuger in Schritt b) wird kultiviert unter Bedingungen, die der Expression der Heterodimerkonstruktion des besagten genetisch verketteten Proteins angemessen sind;
- die Trennung von nicht-löslichen Proteinen in Schritt d) wird löslich gemacht in einer Pufferlösung, welche Urea als chaotropen Wirkstoff und Dithiothreithol als Reduktionsmittel enthält,
- die Renaturierung des Heterodimärs in Schritt e) ist eine oxidative Renaturierung in einer Pufferlösung, welche eine mittlere Konzentration von Urea bei Vorhandensein von Dithiothreitol (DTT) als Reduktionsmittel und oxidiertem Glutathion (GSSG) als Oxidationsmittel enthält;
- das renaturierte Zielprotein in Schritt f) wird aufgereinigt durch eine sequentielle Anionenaustauschchromatographie mit einer zusätzlichen Mixed-Mode-Chromatographie und einer Kationenaustauschchromatographie.

8. Verfahren zur Herstellung nach Anspruch 7 zeichnet sich dadurch aus, dass die besagte Renaturierung des heterodimären Proteins in einer Pufferlösung mit einem pH-Wert von 7,5 - 8,5 durchgeführt wird, wobei diese 2M Urea bei Vorhandensein von Dithiothreitol und oxidiertem Glutathion im molaren Verhältnis von DTT:GSSG 5- 1:1 -5 enthält; vorzugsweise in einer Pufferlösung mit einem pH-Wert von 8,0 und DTT:GSSG im molaren Verhältnis von 1:5.

9. Verfahren zur Herstellung nach Anspruch 8 ist **dadurch gekennzeichnet, dass** in Schritt f) die besagte Anionenaustauschchromatographie auf DEAE-Sepharose FF Sorbents bei einem pH-Wert von 7,0 - 8,5, vorzugsweise bei 7,5, durchgeführt wird, wobei schrittweise der Elutionsmodus angewendet wird; die Mixed-Mode-Chromatographie wird auf CHT Hydroxylapatit Sorbents mit einem pH-Wert von 6,5 - 7.5, vorzugsweise 7,2 durchgeführt; die Kationenaustauschchromatographie ist auf SP-Sepharose FF Sorbents bei einem pH-Wert von 4,5 - 5,2, vorzugsweise 4,7, durchzuführen; die gewonnene Proteinlösung ist konzentriert und wird in einer essigsauren Säure/NaOH-Pufferlösung gelagert.

10. Verfahren zur Herstellung nach Anspruch 9, ist **gekennzeichnet dadurch, dass** in Schritt a) die besagte DNA, die das genetisch verkettete Zielprotein nach Ansprüche 1-4 kodiert, in das Expressionsplasmid pET21b eingefügt wird und ausgewählte Plasmid umgewandelt werden zu Bakterien *E. coli BL21(DE3)*-Stammerzeugern, und in Schritt b) der besagte Erzeuger kultiviert wird in einem LB-Medium bei 37°C bis zur optischen Dichte (OD₆₀₀ₙₘ) 0,8-1,2, wobei Isopropyl-p-D-thiogalactopyranosid (IPTG) als Induktor genutzt wird.

11. Verkettetes SCF-GCSF-Protein, das durch die Verfahren in Anspruch 10 gewonnen wurde, für die therapeutische Anwendung.

## Revendications

1. Protéine fusionnée, qui est un hétérodimère de facteur de stimulation des colonies de granulocytes avec un autre facteur de croissance, de formule générale (I)
X-L-GCSF (I)
où L est la séquence de liaison SGLEA-(EAAAK)ₘ -ALEA-(EAAAK)ₘ -ALEGS, où m=2-8, X désigne SCF, et les séquences d'acides aminés des chaînes monomères sont identiques au moins à 95 % aux séquences d'acides aminés des protéines natives correspondantes.

2. Protéine fusionnée selon la revendication 1, où la séquence de liaison L est représentée par SEQ ID NO: 5.

3. Protéine fusionnée selon l'une quelconque des revendications 1 à 2, où les séquences d'acides aminés des chaînes monomères correspondent à celles des protéines natives, formant une protéine fusionnée représentée par SEQ ID NO: 10.

4. Protéine fusionnée selon l'une quelconque des revendications précédentes, **caractérisée par** une demi-vie de circulation prolongée *in vivo* et une activité synergique.

5. ADN recombinant encodant une protéine fusionnée selon les revendications 1 à 4.

6. ADN recombinant selon la revendication 5, où la séquence codante est représentée par SEQ ID NO: 15.

7. Procédé de préparation d'une protéine hétérodimère de facteur de stimulation des colonies de granulocytes et du facteur des cellules souches selon l'une des revendications 1 à 4, comprenant les étapes suivantes:
a) construction d'ADN, encodant la protéine cible génétiquement fusionnée et préparation du producteur bactérienne ;
b) la culture de la souche de producteur, dont les cellules constituent une séquence d'ADN, encodant la protéine cible dans un milieu de culture approprié ;
c) lyse des microorganismes du producteur et la séparation de la fraction de protéines cibles insolubles ;
d) solubilisation de la fraction des protéines insolubles ;
e) renaturation de la protéine cible ;
f) purification de la protéine cible,
**caractérisé en ce que** la protéine cible est une protéine génétiquement fusionnée selon les revendications 1 à 4 ;
- le producteur de microorganisme à l'étape b) est cultivé dans des conditions appropriées pour l'expression de la construction hétérodimère de ladite protéine génétiquement fusionnée.
- la fraction des protéines insolubles à l'étape d) est solubilisée dans une solution tampon, contenant de l'urée comme agent chaotropique et du dithiothréitol comme agent réducteur ;
- la renaturation de l'hétérodimère à l'étape e) est une renaturation oxydative dans une solution tampon contenant une concentration d'urée intermédiaire en présence de dithiothréitol (DTT) en tant qu'agent réducteur et de glutathion oxydé (GSSG) en tant qu'agent oxydant ;
- la protéine cible renaturée à l'étape f) est purifiée par chromatographie séquentielle d'échange d'anions, une chromatographie de mode mixte et une chromatographie d'échange de cations.

8. Procédé selon la revendication 7, **caractérisé en ce que** ladite renaturation de la protéine hétérodimère est effectuée dans une solution tampon de pH 7,5 - 8,5 contenant 2M d'urée en présence de dithiothréitol et de glutathion oxydé dans un rapport molaire DTT :GSSG 5-1:1-5 ; de préférence dans une solution tampon à pH 8,0 et un rapport molaire DTT :GSSG 1:5.

9. Le procédé selon la revendication 8, **caractérisé en ce qu'**à l'étape f) ladite chromatographie d'échange d'anions est effectuée sur un sorbant DEAE-Sepharose FF à pH 7,0-8,5, de préférence à pH 7,5, en appliquant un mode d'élution par étapes ; ladite chromatographie en mode mixte est effectuée sur un sorbant d'hydroxyapatite CHT à pH 6,5-7,5, de préférence à pH 7,2 ; ladite chromatographie d'échange de cations est effectuée sur un sorbant SP-Sepharose FF à pH 4,5-5,2, de préférence à pH 4,7 ; la solution de protéines obtenue est concentrée et stockée dans une solution tampon d'acide acétique / NaOH.

10. Le procédé selon la revendication 9, **caractérisé en ce qu'**à l'étape a) ledit ADN, encodant la protéine cible génétiquement fusionnée selon les revendications 1 à 4, est inséré dans le plasmide d'expression pET21b et les plasmides sélectionnés sont transformés en producteur bactérienne *E. coli BL21 (DE3),* et à l'étape b) ledit producteur est cultivé dans un milieu LB à une température de 37°C jusqu'à une densité optique (OD₆₀₀ₙₘ) de 0,8-1,2, en utilisant de l'isopropyle-β-D-thiogalactopyranoside (IPTG) comme inducteur.

11. Protéine fusionnée SCF-GCSF, obtenue par la méthode de la revendication 10 pour une utilisation en thérapie.


### SEQUENCE LISTING

<110> UAB Profarma
<120> Fused proteins of granulocyte colony-stimulating factor with other partners of growth factor, preferably with stem cell factor, and method of preparation thereof
<130> 2804
<150> LT2013 104
   <151> 2013-09-27
<160> 15
<170> PatentIn version 3.5
<210> 1
   <211> 11
   <212> PRT
   <213> Artificial Sequence
<220>
   <223> Linker sequence
<400> 1
   Ser Gly Gly Gly Gly Ser Gly Gly Gly Gly Ser
   1               5                   10
<210> 2
   <211> 16
   <212> PRT
   <213> Artificial Sequence
<220>
   <223> Linker sequence
<400> 2
   Ser Gly Gly Gly Gly Ser Gly Gly Gly Gly Ser Gly Gly Gly Gly Ser
   1               5                   10                  15
<210> 3
   <211> 26
   <212> PRT
   <213> Artificial Sequence
<220>
   <223> Linker sequence
<400> 3
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 4
   <211> 36
   <212> PRT
   <213> Artificial Sequence
<220>
   <223> Linker sequence
<400> 4
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser Gly Gly Gly Gly Ser Gly
               20                  25                  30
   Gly Gly Gly Ser
           35
<210> 5
   <211> 54
   <212> PRT
   <213> Artificial Sequence
<220>
   <223> Linker sequence
<400> 5
   Ser Gly Leu Glu Ala Glu Ala Ala Ala Lys Glu Ala Ala Ala Lys Glu
   1               5                   10                  15
   Ala Ala Ala Lys Glu Ala Ala Ala Lys Ala Leu Glu Ala Glu Ala Ala
               20                  25                  30
   Ala Lys Glu Ala Ala Ala Lys Glu Ala Ala Ala Lys Glu Ala Ala Ala
           35                  40                  45
   Lys Ala Leu Glu Gly Ser
       50
<210> 6
   <211> 351
   <212> PRT
   <213> Artificial Sequence
<220>
   <223> Heterodimeric protein
<400> 6
   Met Glu Gly Ile Cys Arg Asn Arg Val Thr Asn Asn Val Lys Asp Val
   1               5                   10                  15
   Thr Lys Leu Val Ala Asn Leu Pro Lys Asp Tyr Met Ile Thr Leu Lys
               20                  25                  30
   Tyr Val Pro Gly Met Asp Val Leu Pro Ser His Cys Trp Ile Ser Glu
           35                  40                  45
   Met Val Val Gln Leu Ser Asp Ser Leu Thr Asp Leu Leu Asp Lys Phe
       50                  55                  60
   Ser Asn Ile Ser Glu Gly Leu Ser Asn Tyr Ser Ile Ile Asp Lys Leu
   65                  70                  75                  80
   Val Asn Ile Val Asp Asp Leu Val Glu Cys Val Lys Glu Asn Ser Ser
                   85                  90                  95
   Lys Asp Leu Lys Lys Ser Phe Lys Ser Pro Glu Pro Arg Leu Phe Thr
               100                 105                 110
   Pro Glu Glu Phe Phe Arg Ile Phe Asn Arg Ser Ile Asp Ala Phe Lys
           115                 120                 125
   Asp Phe Val Val Ala Ser Glu Thr Ser Asp Cys Val Val Ser Ser Thr
       130                 135                 140
   Leu Ser Pro Glu Lys Asp Ser Arg Val Ser Val Thr Lys Pro Phe Met
   145                 150                 155                 160
   Leu Pro Pro Val Ala Ala Ser Gly Gly Gly Gly Ser Gly Gly Gly Gly
                   165                 170                 175
   Ser Thr Pro Leu Gly Pro Ala Ser Ser Leu Pro Gln Ser Phe Leu Leu
               180                 185                 190
   Lys Cys Leu Glu Gln Val Arg Lys Ile Gln Gly Asp Gly Ala Ala Leu
           195                 200                 205
   Gln Glu Lys Leu Cys Ala Thr Tyr Lys Leu Cys His Pro Glu Glu Leu
       210                 215                 220
   Val Leu Leu Gly His Ser Leu Gly Ile Pro Trp Ala Pro Leu Ser Ser
   225                 230                 235                 240
   Cys Pro Ser Gln Ala Leu Gln Leu Ala Gly Cys Leu Ser Gln Leu His
                   245                 250                 255
   Ser Gly Leu Phe Leu Tyr Gln Gly Leu Leu Gln Ala Leu Glu Gly Ile
               260                 265                 270
   Ser Pro Glu Leu Gly Pro Thr Leu Asp Thr Leu Gln Leu Asp Val Ala
           275                 280                 285
   Asp Phe Ala Thr Thr Ile Trp Gln Gln Met Glu Glu Leu Gly Met Ala
       290                 295                 300
   Pro Ala Leu Gln Pro Thr Gln Gly Ala Met Pro Ala Phe Ala Ser Ala
   305                 310                 315                 320
   Phe Gln Arg Arg Ala Gly Gly Val Leu Val Ala Ser His Leu Gln Ser
                   325                 330                 335
   Phe Leu Glu Val Ser Tyr Arg Val Leu Arg His Leu Ala Gln Pro
               340                 345                 350
<210> 7
   <211> 356
   <212> PRT
   <213> Artificial sequence
<220>
   <223> Heterodimeric protein
<400> 7
   Met Glu Gly Ile Cys Arg Asn Arg Val Thr Asn Asn Val Lys Asp Val
   1               5                   10                  15
   Thr Lys Leu Val Ala Asn Leu Pro Lys Asp Tyr Met Ile Thr Leu Lys
               20                  25                  30
   Tyr Val Pro Gly Met Asp Val Leu Pro Ser His Cys Trp Ile Ser Glu
           35                  40                  45
   Met Val Val Gln Leu Ser Asp Ser Leu Thr Asp Leu Leu Asp Lys Phe
       50                  55                  60
   Ser Asn Ile Ser Glu Gly Leu Ser Asn Tyr Ser Ile Ile Asp Lys Leu
   65                  70                  75                  80
   Val Asn Ile Val Asp Asp Leu Val Glu Cys Val Lys Glu Asn Ser Ser
                   85                  90                  95
   Lys Asp Leu Lys Lys Ser Phe Lys Ser Pro Glu Pro Arg Leu Phe Thr
               100                 105                 110
   Pro Glu Glu Phe Phe Arg Ile Phe Asn Arg Ser Ile Asp Ala Phe Lys
           115                 120                 125
   Asp Phe Val Val Ala Ser Glu Thr Ser Asp Cys Val Val Ser Ser Thr
       130                 135                 140
   Leu Ser Pro Glu Lys Asp Ser Arg Val Ser Val Thr Lys Pro Phe Met
   145                 150                 155                 160
   Leu Pro Pro Val Ala Ala Ser Gly Gly Gly Gly Ser Gly Gly Gly Gly
                   165                 170                 175
   Ser Gly Gly Gly Gly Ser Thr Pro Leu Gly Pro Ala Ser Ser Leu Pro
               180                 185                 190
   Gln Ser Phe Leu Leu Lys Cys Leu Glu Gln Val Arg Lys Ile Gln Gly
           195                 200                 205
   Asp Gly Ala Ala Leu Gln Glu Lys Leu Cys Ala Thr Tyr Lys Leu Cys
   210 215 220
   His Pro Glu Glu Leu Val Leu Leu Gly His Ser Leu Gly Ile Pro Trp
   225 230 235 240
   Ala Pro Leu Ser Ser Cys Pro Ser Gln Ala Leu Gln Leu Ala Gly Cys
   245 250 255
   Leu Ser Gln Leu His Ser Gly Leu Phe Leu Tyr Gln Gly Leu Leu Gln
   260 265 270
   Ala Leu Glu Gly Ile Ser Pro Glu Leu Gly Pro Thr Leu Asp Thr Leu
   275 280 285
   Gln Leu Asp Val Ala Asp Phe Ala Thr Thr Ile Trp Gln Gln Met Glu
   290 295 300
   Glu Leu Gly Met Ala Pro Ala Leu Gln Pro Thr Gln Gly Ala Met Pro
   305 310 315 320
   Ala Phe Ala Ser Ala Phe Gln Arg Arg Ala Gly Gly Val Leu Val Ala
   325 330 335
   Ser His Leu Gln Ser Phe Leu Glu Val Ser Tyr Arg Val Leu Arg His
   340 345 350
   Leu Ala Gln Pro
   355
<210> 8
   <211> 366
   <212> PRT
   <213> Artificial Sequence
<220>
   <223> Heterodimeric protein
<400> 8
   Met Glu Gly Ile Cys Arg Asn Arg Val Thr Asn Asn Val Lys Asp Val
   1 5 10 15
   Thr Lys Leu Val Ala Asn Leu Pro Lys Asp Tyr Met Ile Thr Leu Lys
   20 25 30
   Tyr Val Pro Gly Met Asp Val Leu Pro Ser His Cys Trp Ile Ser Glu
   35 40 45
   Met Val Val Gln Leu Ser Asp Ser Leu Thr Asp Leu Leu Asp Lys Phe
   50 55 60
   Ser Asn Ile Ser Glu Gly Leu Ser Asn Tyr Ser Ile Ile Asp Lys Leu
   65 70 75 80
   Val Asn Ile Val Asp Asp Leu Val Glu Cys Val Lys Glu Asn Ser Ser
   85 90 95
   Lys Asp Leu Lys Lys Ser Phe Lys Ser Pro Glu Pro Arg Leu Phe Thr
   100 105 110
   Pro Glu Glu Phe Phe Arg Ile Phe Asn Arg Ser Ile Asp Ala Phe Lys
   115 120 125
   Asp Phe Val Val Ala Ser Glu Thr Ser Asp Cys Val Val Ser Ser Thr
   130 135 140
   Leu Ser Pro Glu Lys Asp Ser Arg Val Ser Val Thr Lys Pro Phe Met
   145 150 155 160
   Leu Pro Pro Val Ala Ala Ser Gly Gly Gly Gly Ser Gly Gly Gly Gly
   165 170 175
   Ser Gly Gly Gly Gly Ser Gly Gly Gly Gly Ser Gly Gly Gly Gly Ser
   180 185 190
   Thr Pro Leu Gly Pro Ala Ser Ser Leu Pro Gln Ser Phe Leu Leu Lys
   195 200 205
   Cys Leu Glu Gln Val Arg Lys Ile Gln Gly Asp Gly Ala Ala Leu Gln
   210 215 220
   Glu Lys Leu Cys Ala Thr Tyr Lys Leu Cys His Pro Glu Glu Leu Val
   225 230 235 240
   Leu Leu Gly His Ser Leu Gly Ile Pro Trp Ala Pro Leu Ser Ser Cys
   245 250 255
   Pro Ser Gln Ala Leu Gln Leu Ala Gly Cys Leu Ser Gln Leu His Ser
   260 265 270
   Gly Leu Phe Leu Tyr Gln Gly Leu Leu Gln Ala Leu Glu Gly Ile Ser
   275 280 285
   Pro Glu Leu Gly Pro Thr Leu Asp Thr Leu Gln Leu Asp Val Ala Asp
   290 295 300
   Phe Ala Thr Thr Ile Trp Gln Gln Met Glu Glu Leu Gly Met Ala Pro
   305 310 315 320
   Ala Leu Gln Pro Thr Gln Gly Ala Met Pro Ala Phe Ala Ser Ala Phe
   325 330 335
   Gln Arg Arg Ala Gly Gly Val Leu Val Ala Ser His Leu Gln Ser Phe
   340 345 350
   Leu Glu Val Ser Tyr Arg Val Leu Arg His Leu Ala Gln Pro
   355 360 365
<210> 9
   <211> 376
   <212> PRT
   <213> Artificial Sequence
<220>
   <223> Heterodimeric protein
<400> 9
   Met Glu Gly Ile Cys Arg Asn Arg Val Thr Asn Asn Val Lys Asp Val
   1 5 10 15
   Thr Lys Leu Val Ala Asn Leu Pro Lys Asp Tyr Met Ile Thr Leu Lys
   20 25 30
   Tyr Val Pro Gly Met Asp Val Leu Pro Ser His Cys Trp Ile Ser Glu
   35 40 45
   Met Val Val Gln Leu Ser Asp Ser Leu Thr Asp Leu Leu Asp Lys Phe
   50 55 60
   Ser Asn Ile Ser Glu Gly Leu Ser Asn Tyr Ser Ile Ile Asp Lys Leu
   65 70 75 80
   Val Asn Ile Val Asp Asp Leu Val Glu Cys Val Lys Glu Asn Ser Ser
   85 90 95
   Lys Asp Leu Lys Lys Ser Phe Lys Ser Pro Glu Pro Arg Leu Phe Thr
   100 105 110
   Pro Glu Glu Phe Phe Arg Ile Phe Asn Arg Ser Ile Asp Ala Phe Lys
   115 120 125
   Asp Phe Val Val Ala Ser Glu Thr Ser Asp Cys Val Val Ser Ser Thr
   130 135 140
   Leu Ser Pro Glu Lys Asp Ser Arg Val Ser Val Thr Lys Pro Phe Met
   145 150 155 160
   Leu Pro Pro Val Ala Ala Ser Gly Gly Gly Gly Ser Gly Gly Gly Gly
   165 170 175
   Ser Gly Gly Gly Gly Ser Gly Gly Gly Gly Ser Gly Gly Gly Gly Ser
   180 185 190
   Gly Gly Gly Gly Ser Gly Gly Gly Gly Ser Thr Pro Leu Gly Pro Ala
   195 200 205
   Ser Ser Leu Pro Gln Ser Phe Leu Leu Lys Cys Leu Glu Gln Val Arg
   210 215 220
   Lys Ile Gln Gly Asp Gly Ala Ala Leu Gln Glu Lys Leu Cys Ala Thr
   225 230 235 240
   Tyr Lys Leu Cys His Pro Glu Glu Leu Val Leu Leu Gly His Ser Leu
   245 250 255
   Gly Ile Pro Trp Ala Pro Leu Ser Ser Cys Pro Ser Gln Ala Leu Gln
   260 265 270
   Leu Ala Gly Cys Leu Ser Gln Leu His Ser Gly Leu Phe Leu Tyr Gln
   275 280 285
   Gly Leu Leu Gln Ala Leu Glu Gly Ile Ser Pro Glu Leu Gly Pro Thr
   290 295 300
   Leu Asp Thr Leu Gln Leu Asp Val Ala Asp Phe Ala Thr Thr Ile Trp
   305 310 315 320
   Gln Gln Met Glu Glu Leu Gly Met Ala Pro Ala Leu Gln Pro Thr Gln
   325 330 335
   Gly Ala Met Pro Ala Phe Ala Ser Ala Phe Gln Arg Arg Ala Gly Gly
   340 345 350
   Val Leu Val Ala Ser His Leu Gln Ser Phe Leu Glu Val Ser Tyr Arg
   355 360 365
   Val Leu Arg His Leu Ala Gln Pro
   370 375
<210> 10
   <211> 394
   <212> PRT
   <213> Artificial sequence
<220>
   <223> Heterodimeric protein
<400> 10
   Met Glu Gly Ile Cys Arg Asn Arg Val Thr Asn Asn Val Lys Asp Val
   1               5                   10                  15
   Thr Lys Leu Val Ala Asn Leu Pro Lys Asp Tyr Met Ile Thr Leu Lys
               20                  25                  30
   Tyr Val Pro Gly Met Asp Val Leu Pro Ser His Cys Trp Ile Ser Glu
           35                  40                  45
   Met Val Val Gln Leu Ser Asp Ser Leu Thr Asp Leu Leu Asp Lys Phe
       50                  55                  60
   Ser Asn Ile Ser Glu Gly Leu Ser Asn Tyr Ser Ile Ile Asp Lys Leu
   65                  70                  75                  80
   Val Asn Ile Val Asp Asp Leu Val Glu Cys Val Lys Glu Asn Ser Ser
                   85                  90                  95
   Lys Asp Leu Lys Lys Ser Phe Lys Ser Pro Glu Pro Arg Leu Phe Thr
               100                 105                 110
   Pro Glu Glu Phe Phe Arg Ile Phe Asn Arg Ser Ile Asp Ala Phe Lys
           115                 120                 125
   Asp Phe Val Val Ala Ser Glu Thr Ser Asp Cys Val Val Ser Ser Thr
       130                 135                 140
   Leu Ser Pro Glu Lys Asp Ser Arg Val Ser Val Thr Lys Pro Phe Met
   145                 150                 155                 160
   Leu Pro Pro Val Ala Ala Ser Gly Leu Glu Ala Glu Ala Ala Ala Lys
                   165                 170                 175
   Glu Ala Ala Ala Lys Glu Ala Ala Ala Lys Glu Ala Ala Ala Lys Ala
               180                 185                 190
   Leu Glu Ala Glu Ala Ala Ala Lys Glu Ala Ala Ala Lys Glu Ala Ala
           195                 200                 205
   Ala Lys Glu Ala Ala Ala Lys Ala Leu Glu Gly Ser Thr Pro Leu Gly
       210                 215                 220
   Pro Ala Ser Ser Leu Pro Gln Ser Phe Leu Leu Lys Cys Leu Glu Gln
   225                 230                 235                 240
   Val Arg Lys Ile Gln Gly Asp Gly Ala Ala Leu Gln Glu Lys Leu Cys
                   245                 250                 255
   Ala Thr Tyr Lys Leu Cys His Pro Glu Glu Leu Val Leu Leu Gly His
                   260                 265                 270
   Ser Leu Gly Ile Pro Trp Ala Pro Leu Ser Ser Cys Pro Ser Gln Ala
               275                 280                 285
   Leu Gln Leu Ala Gly Cys Leu Ser Gln Leu His Ser Gly Leu Phe Leu
       290                 295                 300
   Tyr Gln Gly Leu Leu Gln Ala Leu Glu Gly Ile Ser Pro Glu Leu Gly
   305                 310                 315                 320
   Pro Thr Leu Asp Thr Leu Gln Leu Asp Val Ala Asp Phe Ala Thr Thr
                   325                 330                 335
   Ile Trp Gln Gln Met Glu Glu Leu Gly Met Ala Pro Ala Leu Gln Pro
               340                 345                 350
   Thr Gln Gly Ala Met Pro Ala Phe Ala Ser Ala Phe Gln Arg Arg Ala
           355                 360                 365
   Gly Gly Val Leu Val Ala Ser His Leu Gln Ser Phe Leu Glu Val Ser
       370                 375                 380
   Tyr Arg Val Leu Arg His Leu Ala Gln Pro
   385                 390
<210> 11
   <211> 1056
   <212> DNA
   <213> Artificial Sequence
<220>
   <223> coding sequence
<400> 11 tcgtaccgcg ttctacgcca ccttgcgcag ccgtaa 1056
<210> 12
   <211> 1071
   <212> DNA
   <213> Artificial Sequence
<220>
   <223> Coding sequence
<400> 12
   atggaaggga tctgccgtaa tcgtgtgact aataatgtaa aagacgtcac taaattggtg     60
   gcaaatcttc caaaagacta catgatcacc ctcaaatatg tcccggggat ggatgttttg    120
   ccaagtcatt gttggataag cgagatggta gtacaattgt cagacagctt gactgatctt    180
   ctggacaagt tttcaaatat ttctgaaggc ttgagtaatt attccatcat agacaaactt    240
   gtgaatatag tcgatgacct tgtggagtgc gtcaaagaaa actcatctaa ggatctaaaa    300
   aaatcattca agagcccaga accccgtctc tttactcctg aagaattctt tcgtattttt    360
   aatcgttcca ttgatgcctt caaggacttt gtagtggcat ctgaaactag tgattgtgtg    420
   gtttcttcaa cattaagtcc tgagaaagat tcccgtgtca gtgtcacaaa accatttatg    480
   ttaccccctg ttgcagcctc cggaggtggt ggttctggtg gtggtggttc tggtggtggt    540
   ggatccacac ctttaggacc tgctagctcc ctgccccaga gcttcctgct caagtgctta    600
   gagcaagtga ggaagatcca gggcgatggc gcagcgctcc aggagaagct gtgtgccacc    660
   tacaagctgt gccaccccga ggagctggtg ctgctcggac actctctggg catcccctgg    720
   gctcccctga gctcctgccc cagccaggcc ctgcagctgg caggctgctt gagccaactc    780
   catagcggcc ttttcctcta ccaggggctc ctgcaggccc tggaagggat atcccccgag    840
   ttgggtccca ccttggacac actgcagctg gacgtcgccg actttgccac caccatctgg    900
   cagcagatgg aagaactggg aatggcccct gccctgcagc ccacccaggg tgccatgccg    960
   gccttcgcct ctgctttcca gcgccgggca ggaggggtcc tggttgctag ccatctgcag   1020
   agcttcctgg aggtgtcgta ccgcgttcta cgccaccttg cgcagccgta a            1071
<210> 13
   <211> 1101
   <212> DNA
   <213> Artificial sequence
<220>
   <223> coding sequence
<400> 13
   atggaaggga tctgccgtaa tcgtgtgact aataatgtaa aagacgtcac taaattggtg     60
   gcaaatcttc caaaagacta catgatcacc ctcaaatatg tcccggggat ggatgttttg    120
   ccaagtcatt gttggataag cgagatggta gtacaattgt cagacagctt gactgatctt    180
   ctggacaagt tttcaaatat ttctgaaggc ttgagtaatt attccatcat agacaaactt    240
   gtgaatatag tcgatgacct tgtggagtgc gtcaaagaaa actcatctaa ggatctaaaa    300
   aaatcattca agagcccaga accccgtctc tttactcctg aagaattctt tcgtattttt 360
   aatcgttcca ttgatgcctt caaggacttt gtagtggcat ctgaaactag tgattgtgtg 420
   gtttcttcaa cattaagtcc tgagaaagat tcccgtgtca gtgtcacaaa accatttatg 480
   ttaccccctg ttgcagcctc cggaggtggt ggttctggtg gtggtggttc tggtggtggt 540
   ggttctggtg gtggtggttc tggtggtggt ggatccacac ctttaggacc tgctagctcc 600
   ctgccccaga gcttcctgct caagtgctta gagcaagtga ggaagatcca gggcgatggc 660
   gcagcgctcc aggagaagct gtgtgccacc tacaagctgt gccaccccga ggagctggtg 720
   ctgctcggac actctctggg catcccctgg gctcccctga gctcctgccc cagccaggcc 780
   ctgcagctgg caggctgctt gagccaactc catagcggcc ttttcctcta ccaggggctc 840
   ctgcaggccc tggaagggat atcccccgag ttgggtccca ccttggacac actgcagctg 900
   gacgtcgccg actttgccac caccatctgg cagcagatgg aagaactggg aatggcccct 960
   gccctgcagc ccacccaggg tgccatgccg gccttcgcct ctgctttcca gcgccgggca 1020
   ggaggggtcc tggttgctag ccatctgcag agcttcctgg aggtgtcgta ccgcgttcta 1080
   cgccaccttg cgcagccgta a 1101
<210> 14
   <211> 1128
   <212> DNA
   <213> Artificial Sequence
<220>
   <223> coding sequence
<400> 14
   atggaaggga tctgccgtaa tcgtgtgact aataatgtaa aagacgtcac taaattggtg 60
   gcaaatcttc caaaagacta catgatcacc ctcaaatatg tcccggggat ggatgttttg 120
   ccaagtcatt gttggataag cgagatggta gtacaattgt cagacagctt gactgatctt 180
   ctggacaagt tttcaaatat ttctgaaggc ttgagtaatt attccatcat agacaaactt 240
   gtgaatatag tcgatgacct tgtggagtgc gtcaaagaaa actcatctaa ggatctaaaa 300
   aaatcattca agagcccaga accccgtctc tttactcctg aagaattctt tcgtattttt 360
   aatcgttcca ttgatgcctt caaggacttt gtagtggcat ctgaaactag tgattgtgtg 420
   gtttcttcaa cattaagtcc tgagaaagat tcccgtgtca gtgtcacaaa accatttatg 480
   ttaccccctg ttgcagcctc cggaggtggt ggttctggtg gtggtggttc tggtggtggt 540
   ggttctggtg gtggtggttc tggtggtggt ggttctggtg gtggtggttc tggtggtggt 600
   ggatccacac ctttaggacc tgctagctcc ctgccccaga gcttcctgct caagtgctta 660
   gagcaagtga ggaagatcca gggcgatggc gcagcgctcc aggagaagct gtgtgccacc 720
   tacaagctgt gccaccccga ggagctggtg ctgctcggac actctctggg catcccctgg 780
   gctcccctga gctcctgccc cagccaggcc ctgcagctgg caggctgctt gagccaactc 840
   catagcggcc ttttcctcta ccaggggctc ctgcaggccc tggaagggat atcccccgag 900
   ttgggtccca ccttggacac actgcagctg gacgtcgccg actttgccac caccatctgg 960
   cagcagatgg aagaactggg aatggcccct gccctgcagc ccacccaggg tgccatgccg 1020
   gccttcgcct ctgctttcca gcgccgggca ggaggggtcc tggttgctag ccatctgcag 1080
   agcttcctgg aggtgtcgta ccgcgttcta cgccaccttg cgcagccg 1128
<210> 15
   <211> 1195
   <212> DNA
   <213> Artificial sequence
<220>
   <223> coding sequence
<400> 15
   catatggaag ggatctgccg taatcgtgtg actaataatg taaaagacgt cactaaattg      60
   gtggcaaatc ttccaaaaga ctacatgatc accctcaaat atgtcccggg gatggatgtt     120
   ttgccaagtc attgttggat aagcgagatg gtagtacaat tgtcagacag cttgactgat     180
   cttctggaca agttttcaaa tatttctgaa ggcttgagta attattccat catagacaaa     240
   cttgtgaata tagtcgatga ccttgtggag tgcgtcaaag aaaactcatc taaggatcta     300
   aaaaaatcat tcaagagccc agaaccccgt ctctttactc ctgaagaatt ctttcgtatt     360
   tttaatcgtt ccattgatgc cttcaaggac tttgtagtgg catctgaaac tagtgattgt     420
   gtggtttctt caacattaag tcctgagaaa gattcccgtg tcagtgtcac aaaaccattt     480
   atgttacccc ctgttgcagc ctccggactt gaggctgaag cagcagctaa agaagcagca     540
   gctaaagaag cagcagctaa agaagcagca gctaaagctc tcgaggctga agcagcagct     600
   aaagaagcag cagctaaaga agcagcagct aaagaagcag cagctaaagc tcttgaggga     660
   tccacacctt taggacctgc tagctccctg ccccagagct tcctgctcaa gtgcttagag     720
   caagtgagga agatccaggg cgatggcgca gcgctccagg agaagctgtg tgccacctac     780
   aagctgtgcc accccgagga gctggtgctg ctcggacact ctctgggcat cccctgggct     840
   cccctgagct cctgccccag ccaggccctg cagctggcag gctgcttgag ccaactccat     900
   agcggccttt tcctctacca ggggctcctg caggccctgg aagggatatc ccccgagttg     960
   ggtcccacct tggacacact gcagctggac gtcgccgact ttgccaccac catctggcag    1020
   cagatggaag aactgggaat ggcccctgcc ctgcagccca cccagggtgc catgccggcc    1080
   ttcgcctctg ctttccagcg ccgggcagga ggggtcctgg ttgctagcca tctgcagagc    1140
   ttcctggagg tgtcgtaccg cgttctacgc caccttgcgc agccgtaata agctt         1195